# EUROPÄISCHE PATENTSCHRIFT

(11) **EP 1 915 093 B1**
(45) Veröffentlichungstag und Bekanntmachung des Hinweises auf die Patenterteilung: **23.02.2011**
(21) Anmeldenummer: 06777848.0
(22) Anmeldetag: 20.07.2006
(51) Int. Cl.: A61B 6/00

(54) **MAMMOGRAPHIEGERÄT MIT EINER DREHBAR ANGEORDNETEN HALTERUNG FÜR ZUMINDEST ZWEI RÖNTGENEMPFÄNGER**
MAMMOGRAPHY APPARATUS COMPRISING A ROTATABLY MOUNTED FIXTURE FOR AT LEAST TWO X-RAY RECEIVERS
APPAREIL DE MAMMOGRAPHIE COMPRENANT UN SUPPORT DISPOSE DE MANIERE ROTATIVE POUR AU MOINS DEUX RECEPTEURS DE RAYONS X

(30) Priorität: 18.08.2005 DE 102005039185
(43) Veröffentlichungstag der Anmeldung: 30.04.2008
(73) Patentinhaber: SIEMENS AKTIENGESELLSCHAFT, 80333 München (DE)
(72) Erfinder: HOFFMANN, Norbert, 90419 Nürnberg (DE); NAGENGAST, Gerhard, 91330 Eggolsheim (DE); RAMSAUER, Martin, 90602 Pyrbaum (DE)
(86) Internationale Anmeldenummer: PCT/EP2006/064434
(87) Internationale Veröffentlichungsnummer: WO 2007/020150

(56) Entgegenhaltungen:
- EP-A1- 0 127 073
- US-A- 5 170 419
- US-A- 5 365 562

## Beschreibung

Die Erfindung bezieht sich auf ein Mammographiegerät mit einer drehbar angeordneten Halterung für zumindest zwei Röntgenempfänger.

Als Mammographiegerät bezeichnet man ein Röntgensystem zur Untersuchung einer Brust eines Patienten oder einer Patientin, insbesondere zur vorbeugenden Untersuchung auf Brustkrebs. Üblicherweise stehen für die Röntgenuntersuchung verschiedene Röntgenempfänger zur Verfügung, zwischen denen für die jeweilige Untersuchung gewechselt werden kann. Die Röntgenempfänger sind jeweils auch ein Objekttisch, auf dem die Brust platziert wird. Es sind sowohl analoge Röntgenempfänger in Form von Röntgenfilmkassetten als auch digitale Röntgenempfänger in Form von digitalen Röntgendetektoren bekannt. Die Röntgenempfänger können - zum Beispiel durch eine lösbare Verbindung der Röntgenempfänger mit einer Halterung des Mammographiegerätes - modular ausgewechselt werden.

Aus der US 5,170,419 ist ein Mammographiegerät mit einem Haltearm bekannt, an dem eine Halterung für zumindest zwei Röntgenempfänger drehbar angeordnet ist; die drehbare Anordnung ist derart ausgebildet, dass jeweils ein Röntgenempfänger in Form einer Röntgenfilmkassette in einer Untersuchungsposition innerhalb eines vorgesehenen Röntgenstrahlungsfeldes, mit dem während der Untersuchung ein Röntgenbild erzeugt wird, positionierbar ist, während die übrigen Röntgenempfänger in jeweils einer Reserveposition außerhalb des Röntgenstrahlungsfeldes verbleiben. Dies ermöglicht ein schnelles Wechseln des Formats des jeweiligen Röntgenempfängers.

Aus der EP 0 127 073 ist ein Mammographiegerät mit zwei wahlweise auf einen Röntgenstrahler ausrichtbaren Aufnahmetischen bekannt, die derart schwenkbar mit dem Mammographiegerät verbunden sind, dass jeweils einer der Aufnahmetische in eine Aufnahmeposition und der andere in einer Parkposition verstellbar sind. Die beiden Aufnahmetische sind derart schwenkbar gelagert, dass sie sich um 180° gegenüberliegen.

Aus der US 5,365,562 ist ein Mammographiegerät bekannt, das eine Signaldetektoreinheit mit einem so genannten Karussell aufweist, an dem eine Vielzahl von Signaldetektorelementen angeordnet sind.

In dem vorgenannten Mammographiegerät muss die Halterung für die Röntgenempfänger den Haltearm ringartig umschließen; dieser konstruktive Aufbau ist aufwändig und erschwert die Wartung.

Der Erfindung liegt die Aufgabe zugrunde, ein Mammographiegerät mit einer drehbaren Halterung für zumindest zwei Röntgenempfänger zu schaffen, das einen verbesserten Geräteaufbau aufweist.

Diese Aufgabe wird ausgehend von einem Mammographiegerät gemäß Oberbegriff des Patentanspruchs 1 durch dessen kennzeichnende Merkmale gelöst; vorteilhafte Ausgestaltungen der Erfindung sind jeweils Gegenstand der Unteransprüche.

Durch die Anordnung der zumindest einen Reserveposition auf der in Röntgenstrahlungsrichtung gelegenen Seite relativ zu dem Haltearm ist es möglich, die Halterung für die Röntgenempfänger konstruktiv einfach mit dem Mammographiegerät zu verbinden. Ein ringartiges Umschließen des Haltearms durch die Halterung, wie in der US 5,170,419, ist nicht notwendig, so dass die Halterung modular an dem Mammographiegerät gewechselt werden kann. Dadurch dass die Reserveposition auf derjenigen Seite des Haltearms angeordnet ist, die sich in Röntgenstrahlungsrichtung relativ zu dem Haltearm befindet, wird die Freiheit zur Positionierung des Röntgenstrahlers zur Erzeugung des Röntgenstrahlungsfeldes durch die Reserveposition nicht beschränkt, wodurch ein kompakterer Geräteaufbau ermöglicht wird. Auf diese Weise ist trotz einer drehbaren Halterung für zumindest zwei Röntgenempfänger in aufwandsarmer Weise ein stabiler Geräteaufbau möglich.

Bei einem Mammographiegerät befinden sich der Röntgenstrahler, die Position der zu untersuchenden Brust sowie der zur Untersuchung vorgesehene Röntgenempfänger in einer linearen Anordnung zueinander; bei der Untersuchung wird von dem Röntgenstrahler die Röntgenstrahlung in Richtung auf den Röntgenempfänger ausgestrahlt, wobei das Röntgenstrahlungsfeld die zu untersuchende Brust durchdringt. Diese Richtung, in der die Röntgenstrahlung ausgestrahlt wird, wird hier und im Folgenden als Röntgenstrahlungsrichtung bezeichnet.

Durch eine lösbare Verbindung der Halterung mit dem Mammographiegerät wird eine besonders hohe Modularität gewährleistet. Dadurch kann die Halterung auf einfache Weise nachgerüstet bzw. ausgetauscht werden. So ist es z.B. möglich, ein im Auslieferungszustand analoges Mammographiegerät nachträglich zu einem digitalen Mammographiegerät durch einen Austausch der Halterung umzurüsten. Es ist auch möglich, das Mammographiegerät zunächst mit einer starren Halterung für jeweils nur einen Röntgenempfänger auszuliefern und diese starre Halterung durch die drehbare Halterung zu einem späteren Zeitpunkt zu ersetzen. Der Verbindungsmechanismus kann z.B. in Form einer Klemm- oder Steckverbindung ausgebildet sein.

Durch eine lösbare Verbindung der Röntgenempfänger mit der Halterung ist es möglich, mit derselben Halterung eine unterschiedliche Auswahl von Röntgenempfängern bereitzustellen. Darüber hinaus wird eine leichte Austauschbarkeit der Röntgenempfänger im Wartungsfall ermöglicht.

Dadurch, dass die durch die Halterung gehaltenen Röntgenempfänger jeweils den gleichen Lateralwinkel zu der Drehachse der Halterung aufweisen, wird in einfacher Weise gewährleistet, dass sämtliche Röntgenempfänger durch die Drehung der Halterung jeweils an der gleichen Untersuchungsposition anordbar sind. Im Fall von Röntgenempfängern mit jeweils einer ebenen Empfängerfläche liegen diese Empfängerflächen tangential auf der Mantelfläche eines imaginären geraden Kreiskegels auf, dessen Symmetrieachse mit der Drehachse der Halterung übereinstimmt, wobei der vorgenannte Winkel dem Öffnungswinkel des Kreiskegels entspricht. Es ist vorgesehen, dass jeder Röntgenempfänger im Wesentlichen in der gleichen Entfernung von der Halterung angeordnet ist, wenn sich der jeweilige Röntgenempfänger in der Untersuchungsposition befindet.

Durch einen Lateralwinkel von 45° wird ein besonders kompakter und Platz sparender Geräteaufbau ermöglicht. Im Fall von lediglich zwei Röntgenempfängern, die auf gegenüberliegenden Seiten relativ zur Halterung angeordnet sind, schließen die beiden Röntgenempfänger bei einem Lateralwinkel von 45° einen Winkel von 90° ein.

Durch eine ungefähr gleiche Radialwinkeldifferenz von benachbarten Röntgenempfängern im Bezug auf die Drehung um die Drehachse der Halterung wird eine besonders gute Raumausnutzung ermöglicht; die Röntgenempfänger in ihrer jeweiligen Reserveposition schränken dabei die Bewegungsfreiheit des Personals bzw. des Patienten besonders wenig ein. Im Fall von lediglich zwei Röntgenempfängern beträgt die Radialwinkeldifferenz gem. dieser Ausgestaltung jeweils 180°, so dass die beiden Röntgenempfänger auf zueinander gegenüberliegenden Seiten der Halterung angeordnet sind.

Durch eine Anordnung der zumindest einen Reserveposition auf der patientenfernen Seite relativ zu der Untersuchungsposition wird eine besonders gute Zugänglichkeit des Patienten gewährleistet. Diese Art der Anordnung kann z.B. dadurch realisiert werden, dass die Halterung an einer patientenfernen Stelle an dem Haltearm befestigt ist.

Konstruktiv einfach ist der Haltearm an einem im Wesentlichen vertikal ausgerichteten Stativ, um die zumindest eine Reserveposition auf der stativnahen Seite relativ zu der Untersuchungsposition angeordnet. Durch die stativnahe Anordnung der zumindest einen Reserveposition werden einerseits ein besonders kompakter Geräteaufbau und andererseits ein besonders stativnaher Schwerpunkt gewährleistet. Zweckmäßig ist der Haltearm an dem Stativ höhenverstellbar.

Konstruktiv aufwandsarm ist der Röntgenstrahler an dem Haltearm angeordnet. Im Fall eines höhenverstellbaren Haltearms sind dadurch in einfacher Weise sowohl der Röntgenstrahler als auch die Röntgenempfänger gemeinsam mit dem Haltearm höhenverstellbar. Zweckmäßig ist die Anordnung aus Haltearm, Röntgenstrahler und Röntgenempfänger in der Untersuchungsposition in Form eines U-Arms ausgebildet.

Durch eine Ausbildung des Haltearms, derart dass dieser um eine im Wesentlichen horizontale Achse drehbar ist, kann die zu untersuchende Brust aus verschiedenen Blickwinkeln untersucht werden; ein um eine horizontale Achse drehbarer Haltearm mit einem Röntgenstrahler und einem Röntgenempfänger ist an sich aus der US 5,170,419 bekannt.

Die Erfindung sowie weitere vorteilhafte Ausgestaltungen der Erfindung gemäß Merkmalen der Unteransprüche werden im Folgenden anhand von schematisch dargestellten Ausführungsbeispielen in der Zeichnung näher erläutert, ohne dass dadurch eine Beschränkung der Erfindung auf diese Ausführungsbeispiele erfolgt; darin zeigen:
- FIG 1: in seitlicher Ansicht ein Mammographiegerät mit einem Haltearm und mit einer unterhalb des Haltearms dreh- bar befestigen Halterung für zwei in einer Ebene an- geordnete Röntgenempfänger;
- FIG 2: in seitlicher Ansicht ein Mammographiegerät gemäß FIG 1, wobei die zwei Röntgenempfänger einen rechten Win- kel bilden;
- FIG 3: in Draufsicht die Halterung für zwei Röntgenempfän- ger;
- FIG 4: in Draufsicht eine Halterung für drei Röntgenempfän- ger.

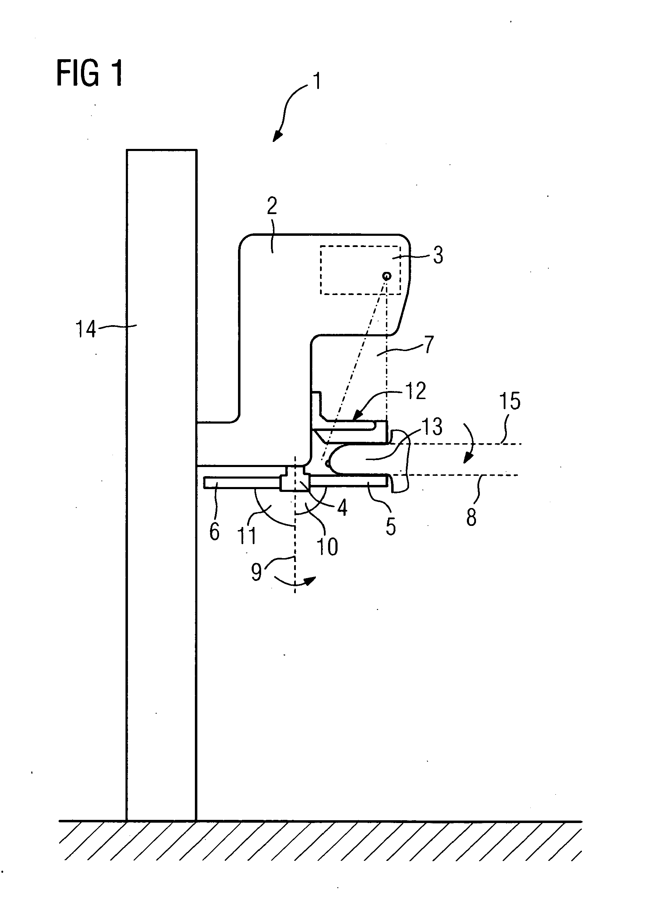

Figur 1 ist kein Ausführungsbeispiel der beanspruchten Enfindung. Figur 1 zeigt ein Mammographiegerät 1 mit einem Haltearm 2, einem an einem oberen Ende des Haltearms 2 angeordneten Röntgenstrahler 3 und einer an einem unteren Ende des Haltearms 2 angeordneten, drehbaren Halterung 4 für zwei Röntgenempfänger 5, 6, derart dass jeweils ein Röntgenempfänger 5 in einer Untersuchungsposition innerhalb des Röntgenstrahlungsfeldes 7 des Röntgenstrahlers 3 drehbar ist, während der verbleibende Röntgenempfänger 6 in einer Reserveposition außerhalb des Röntgenstrahlungsfeldes 7 verbleibt; der eine Röntgenempfänger 5 in der Untersuchungsposition ist mit seiner Empfängeroberfläche im Wesentlichen in Richtung auf den Röntgenstrahler 3 ausgerichtet, während der verbleibende Röntgenempfänger 6 in seiner Reserveposition in der Ebene 8 angeordnet ist, die durch die Empfängeroberfläche des einen Röntgenempfängers 5 in der Untersuchungsposition definiert wird.

In diesem Beispiel handelt es sich um ein Mammographiegerät 1 zur Untersuchung einer Patientin bzw. eines Patienten in einer aufrechten Position, z.B. im Stehen oder im Sitzen. Zur Höhenanpassung an die Lage des jeweiligen Untersuchungsobjektes 13 ist der Haltearm 2 höhenverstellbar an einem Bodenstativ 14 befestigt. Zur Einstellung verschiedener Blickwinkel bei der Untersuchung des Untersuchungsobjektes 13 ist der Haltearm 2 um eine horizontale Schwenkachse 15 schwenkbar. Zur Untersuchung wird das jeweilige Untersuchungsobjekt 13 mit einer auf das Untersuchungsobjekt 13 herabfahrbaren Kompressionsplatte 12 komprimiert.

Die unterhalb des Haltearms 2 angeordnete Halterung 4 ist lösbar mit dem Haltearm 2 verbunden; anstelle der drehbaren Halterung 4 kann auch eine statische Halterung für lediglich einen Röntgenempfänger an dem Haltearm befestigt werden. Die beiden Röntgenempfänger 5, 6 sind lösbar mit der Halterung 4 verbunden, wobei diese Verbindung über einen Steckmechanismus hergestellt wird. Dieser Steckmechanismus ist im betriebsmäßigen Zustand des Mammographiegerätes 1 verriegelt und kann per Knopfdruck zum Austausch eines der beiden Röntgenempfänger 5, 6 mechanisch entriegelt werden.

Die Halterung 4 ist zusammen mit den Röntgenempfängern 5, 6 um eine in der Grundstellung des Haltearms 2 vertikalen Drehachse 9 drehbar, so dass die beiden Röntgenempfänger 5, 6 in ihrer jeweiligen Position vertauscht werden können. Der Röntgenempfänger 5 und der Röntgenempfänger 6 schließen mit der Drehachse 9 einen Lateralwinkel 10 bzw. einen Lateralwinkel 11 von jeweils 90° ein, so dass die beiden Röntgenempfänger 5, 6 in einer Ebene angeordnet sind. Die dargestellte Anordnung der Halterung 4 aus Sicht der jeweiligen Patientin bzw. des jeweiligen Patienten hinter der Kompressionsplatte 12 gewährleistet, dass die Reserveposition auf der patientenfernen Seite relativ zu der Untersuchungsposition stativnah angeordnet ist. Die äußeren Enden der Röntgenempfänger 5, 6 weisen jeweils denselben Abstand zu der Drehachse 9 auf, so dass beide Röntgenempfänger 5, 6 durch die Drehung in dieselbe Untersuchungsposition positionierbar sind.

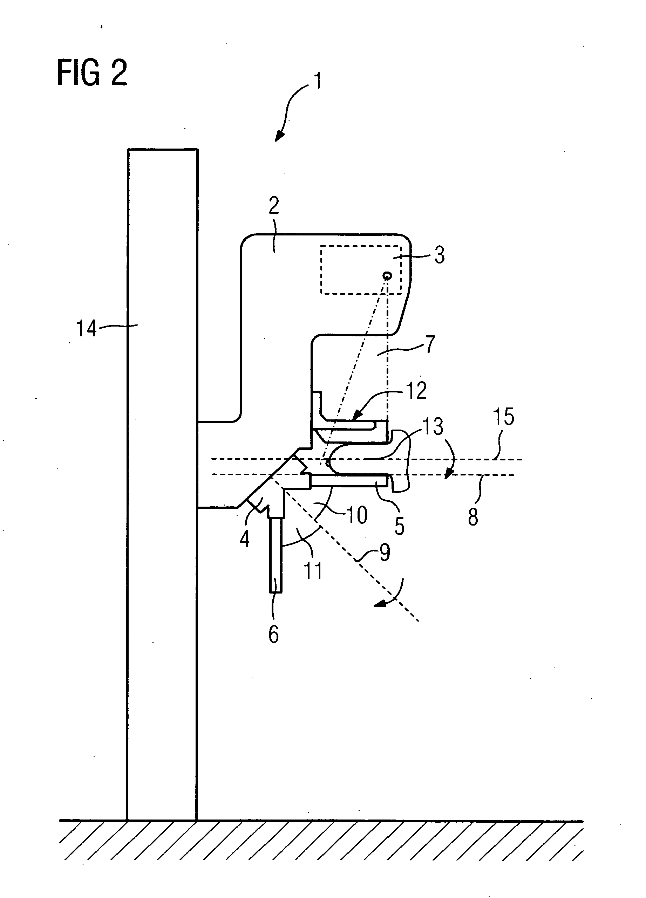

Figur 2 zeigt ein Mammographiegerät 1 gemäß Figur 1, wobei der verbleibende Röntgenempfänger 6 in seiner Reserveposition unterhalb der Ebene 8 angeordnet ist, die durch die Empfängeroberfläche des einen Röntgenempfängers 5 in der Untersuchungsposition definiert wird; in diesem Ausführungsbeispiel betragen die beiden Lateralwinkel 10, 11 jeweils 45°, so dass die beiden Röntgenempfänger 5, 6 zusammen einen rechten Winkel bilden. Während der eine Röntgenempfänger 5 in der Untersuchungsposition auf die Patientin gerichtet ist, deren Brust 13 mit dem Mammographiegerät 1 untersucht wird, ist der verbleibende Röntgenempfänger 5 in der Reserveposition nach unten gerichtet.

Durch diese Anordnung der Halterung 4 und der Röntgenempfänger 5, 6 wird der Schwerpunkt des Haltearms 2 mit den an diesem befestigten Komponenten gegenüber dem in Figur 1 gezeigten Ausführungsbeispiel näher an das Stativ 14 gerückt; diese kompaktere Bauweise ermöglicht einen stabileren Geräteaufbau.

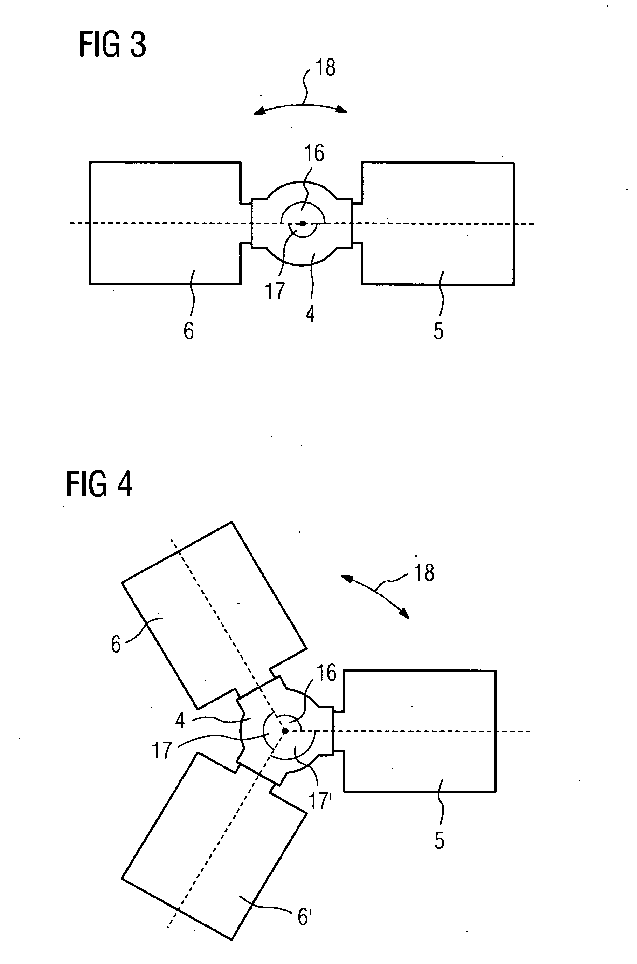

Figur 3 zeigt in Draufsicht eine Halterung 4 gemäß Figur 1 bzw. gemäß Figur 2 mit den zwei Röntgenempfängern 5, 6. Wie in den Figuren 1 und 2 sind die beiden Röntgenempfänger 5, 6 auf gegenüberliegenden Seiten der Halterung 4 angeordnet, so dass die beiden Röntgenempfänger 5, 6 in Drehrichtung 18 einen Radialwinkeldifferenz 16 bzw. 17 von jeweils 180° einschließen.

Figur 4 zeigt eine Halterung 4 gemäß Figur 3 in diesem Ausführungsbeispiel jedoch mit drei Röntgenempfängern 5, 6, 6', die jeweils mit ihren benachbarten Röntgenempfänger einen Radialwinkeldifferenz 16 bzw. 17 bzw. 17' einschließen.

## Patentansprüche

1. Mammographiegerät (1) mit einem Haltearm (2), an dem eine Halterung (4) mit zumindest zwei Röntgenempfängern (5, 6, 6') um eine Drehachse (9) drehbar angeordnet ist, derart dass jeweils ein Röntgenempfänger (5, 6, 6') in einer Untersuchungsposition innerhalb eines vorgesehenen Röntgenstrahlungsfeldes (7) bei einem gleichzeitigen Verbleib der übrigen Röntgenempfänger (5, 6, 6') in jeweils einer Reserveposition außerhalb des Röntgenstrahlungsfeldes (7) positionierbar ist, bei dem
- die Röntgenempfänger (5, 6, 6') mit der Drehachse (9) einen Winkel von 45° einschließen,
- eine Ebene (8) durch die Empfängeroberfläche des einen Röntgenempfängers (5) in der Untersuchungsposition definiert ist,
**dadurch gekennzeichnet, dass** der oder die übrigen Röntgenempfänger (6, 6') in jeweils einer Reserveposition unterhalb der Ebene (8) angeordnet sind.

2. Mammographiegerät (1) nach Anspruch 1, **dadurch gekennzeichnet, dass** die Halterung (4) lösbar mit dem Halterarm (2) verbindbar ist.

3. Mammographiegerät (1) nach Anspruch 2 und/oder 3, **dadurch gekennzeichnet, dass** Röntgenempfänger (5, 6, 6') lösbar mit der Halterung (4) verbindbar ist.

4. Mammographiegerät (1) nach einem der vorhergehenden Ansprüche, **dadurch gekennzeichnet, dass** die Radialwinkeldifferenz (16, 17, 17') von benachbarten Röntgenempfängern (5, 6, 6') in Bezug auf die Drehung um die Drehachse (9) der Halterung (4) jeweils ungefähr gleich ist.

5. Mammographiegerät (1) nach einem der vorhergehenden Ansprüche, **dadurch gekennzeichnet, dass** die zumindest eine Reserveposition auf der patientenfernen Seite relativ zu der Untersuchungsposition angeordnet ist.

6. Mammographiegerät (1) nach einem der vorhergehenden Ansprüche, **dadurch gekennzeichnet, dass** der Haltearm (2) an einem im Wesentlichen vertikal ausgerichteten Stativ (14) und die zumindest eine Reserveposition auf der stativnahen Seite relativ zu der Untersuchungsposition angeordnet sind.

7. Mammographiegerät (1) nach einem der vorhergehenden Ansprüche, **dadurch gekennzeichnet, dass** der Röntgenstrahler (3) an dem Haltearm (2) angeordnet ist.

8. Mammographiegerät (1) nach einem der vorhergehenden Ansprüche, **dadurch gekennzeichnet, dass** der Haltearm (2) um eine im Wesentlichen horizontale Schwenkachse (15) schwenkbar ist.

## Claims

1. Mammography device (1) having a retaining arm (2), on which a mounting (4) having at least two x-ray receivers (5, 6, 6') is rotatably arranged about an axis of rotation (9) such that an x-ray receiver (5, 6, 6') can be positioned in each instance in an examination position within a provided x-ray radiation field (7) while simultaneously retaining the other x-ray receivers (5, 6, 6') in a reserve position outside of the x-ray radiation field (7) in each instance, in which
- the x-ray receivers (5, 6, 6') enclose an angle of 45° with the axis of rotation (9),
- a plane (8) is defined through the receiver surface of the one x-ray receiver (5) in the examination position, **characterised in that**
- the or the other x-ray receiver/s (6, 6') is/are arranged in a reserve position below the plane (8) in each instance.

2. Mammography device (1) according to claim 1, **characterised in that** the mounting (4) can be detachably connected to the retaining arm (2).

3. Mammography device (1) according to claim 2 and/or 3, **characterised in that** the x-ray receiver (5, 6, 6') can be detachably connected to the mounting (4).

4. Mammography device (1) according to one of the preceding claims, **characterised in that** the radial angle difference (16, 17, 17') of adjacent x-ray receivers (5, 6, 6') is in each instance approximately equivalent in respect of the rotation about the axis of rotation (9) of the mounting (4).

5. Mammography device (1) according to one of the preceding claims, **characterised in that** the at least one reserve position is arranged on the side remote from the patient and relative to the examination position.

6. Mammography device (1) according to one of the preceding claims, **characterised in that** the retaining arm (2) is arranged on an essentially vertically aligned stand (14) and the at least one reserve position is arranged on the side near to the stand and relative to the examination position.

7. Mammography device (1) according to one of the preceding claims, **characterised in that** the x-ray emitter (3) is arranged on the retaining arm (2).

8. Mammography device (1) according to one of the preceding claims, **characterised in that** the retaining arm (2) can be pivoted about an essentially horizontal swivel axis (15).

## Revendications

1. Appareil de mammographie (1) pourvu d'un bras de support (2) sur lequel un support (4), comprenant au moins deux récepteurs de rayons X (5, 6, 6'), est disposé de manière rotative autour d'un axe de rotation (9), de telle façon que chaque fois l'un des récepteurs de rayons X (5, 6, 6') puisse être positionnée dans une position d'examen à l'intérieur d'un champ de rayons X (7), pendant que les autres récepteurs de rayons X (5, 6, 6') demeurent chacun dans une position de réserve à l'extérieur du champ de rayons X (7), appareil dans lequel
- les récepteurs de rayons X (5, 6, 6') sous-tendent avec l'axe de rotation (9) un angle de 45°,
- un plan (8) est défini par la surface réceptrice dudit un récepteur de rayons X (5) en position d'examen,
**caractérisé en ce que** le ou les autres récepteurs de rayons X (6, 6') sont disposés chacun dans une position de réserve au-dessous du plan (8).

2. Appareil de mammographie (1) selon la revendication 1, **caractérisé en ce que** le support (4) est susceptible d'être relié de manière amovible au bras de support (2).

3. Appareil de mammographie (1) selon la revendication 2 et/ou 3, **caractérisé en ce que** le récepteur de rayons X (5, 6, 6') est susceptible d'être relié de manière amovible au support (4).

4. Appareil de mammographie (1) selon l'une des revendications précédentes, **caractérisé en ce que** la différence angulaire radiale (16, 17, 17') de récepteurs de rayons X voisins (5, 6, 6'), par rapport à la rotation autour de l'axe de rotation (9) du support (4), est à chaque fois sensiblement la même.

5. Appareil de mammographie (1) selon l'une des revendications précédentes, **caractérisé en ce que** ladite au moins une position de réserve est disposée sur le côté éloigné du patient par rapport à la position d'examen.

6. Appareil de mammographie (1) selon l'une des revendications précédentes, **caractérisé en ce que** le bras de support (2) est disposé sur un pied (14) d'orientation essentiellement verticale, et ladite au moins une position de réserve est disposée sur le côté proche du pied par rapport à la position d'examen.

7. Appareil de mammographie (1) selon l'une des revendications précédentes, **caractérisé en ce que** l'émetteur de rayons X (3) est disposé sur le bras de support (2).

8. Appareil de mammographie (1) selon l'une des revendications précédentes, **caractérisé en ce que** le bras de support (2) est susceptible de pivoter autour d'un axe de pivotement (15) essentiellement horizontal.
